# EUROPEAN PATENT APPLICATION

(11) **EP 4 596 679 A1**
(43) Date of publication of application: **06.08.2025**
(21) Application number: 23872794.5
(22) Date of filing: 14.08.2023
(51) Int. Cl.: C12N 5/078, C12N 5/0783

(54) **NOVEL DUAL CULTURE METHOD FOR CULTURING IMMUNE CELLS AND NATURAL KILLER CELLS, AND USE THEREOF**

(30) Priority: 28.09.2022 KR 20220123845; 18.04.2023 KR 20230050752
(71) Applicant: Forevernk Inc., Seongnam-si, Gyeonggi-do 13449 (KR); Oh, Jung Hoon, Suwon-si, Gyeonggi-do 16330 (KR)
(72) Inventor: BAEK, Young Il, Suwon-si Gyeonggi-do 16330 (KR); OH, Jung Hoon, Suwon-si Gyeonggi-do 16330 (KR)
(74) Representative: dompatent von Kreisler Selting Werner - Partnerschaft von Patent- und Rechtsanwälten mbB
(86) International application number: PCT/KR2023/012028
(87) International publication number: WO 2024/071671

(57) **Abstract**

The present invention relates to a method for the dual culture of immune cells used for immunotherapy, and more specifically, to a dual culture method in which immune cells, e.g., immune cells (PBMCs), natural killer cells, and gamma delta T cells, which are highly effective in treating malignant tumors and the like, are selectively and efficiently amplified and activated by culturing lymphocytes derived from human peripheral blood, wherein the culture efficiency and activity can be increased when immune cells are cultured in combination after applying different stimuli for a predetermined period of time.

## Description

### [Technical Field]

The present invention relates to a method for the dual culture of immune cells used for immunotherapy, and more specifically, to a culture method for selectively and efficiently expanding and activating immune cells that are highly effective in treating malignant tumors, such as immune cells (PBMC), natural killer cells, or gamma delta T cells, by culturing lymphocytes derived from human peripheral blood.

### [Background Art]

Immunotherapy is a method in which the most important immune cells for cancer treatment, such as natural killer cells (NK cells), dendritic cells (DCs), B cells, and T cells, are extracted from the blood of patients and are then grown into immune cells that act strongly against cancer using various stimulants, and then incorporated back into the patient. Immunotherapy has fewer side effects due to use of the patient's own blood and is easier to administer than conventional chemotherapy and thus has been actively researched recently.

NK cells do not attack normal cells that express MHC class I molecules, but mainly attack cells with reduced or defective expression of MHC class I molecules. Therefore, use of allogeneic NK cells in cell therapy for cancer and infections has the advantage of avoiding the side effects of GVH (graft-versus-host) disease. In fact, it was reported by Miller et al. and Rubnitz et al. that, when NK cells were concentrated from fresh peripheral blood mononuclear cells of a normal donor who was a close relative of cancer patients and transplanted into a cancer patient as a recipient, the transplanted NK cells did not cause side effects in the recipient, temporarily engrafted, and maintained cytotoxic activity. However, there is no report on the efficacy of clinical treatment that demonstrates the effectiveness of NK cell transplantation therapy. One of the reasons is that it is impossible to maintain a sufficient number of NK cells in the body of the recipient until the target cells are killed due to the limit on the number of cells that can be recovered from the donor by lymphocyte apheresis.

Therefore, in order to maintain a sufficient number of NK cells in the recipient body until the target cells are killed, such as cancer cells and pathogen-infected cells, frequent repeated transplantation of NK cells is necessary, which is a great burden on the patient. Therefore, a technology for obtaining NK cells sufficient to kill target cells by culturing and amplifying natural killer cells obtained from donors *in vitro* is being developed.

Among the immune cells activated in immunotherapy, natural killer cells are a characteristic type of lymphocytes, namely, large granular lymphocytes (LGL), which have an excellent ability to kill infected viruses and tumor cells, but do not kill most normal cells. The antitumor action of natural killer cells is achieved by necrosis, apoptosis, or simultaneous occurrence of these two mechanisms. NK cells respond to cytokines such as IL-2, IL-12, and interferons. As a result, cytotoxicity, and secretory and proliferative functions are improved. The phenotype of NK cells is CD16 antibody (FcγRIII) and CD56 antibody in humans, and CD16 antibody and CD56 antibody are used as markers for NK cells because they do not have TRC (T-cell receptor complex) on the cell surface.

It is known that these NK cells play an important role in the initial biological defense mechanism and tumor immunity of the human body. In other words, NK cells can kill specific self-cells, allogeneic cells, and even xenogeneic cancer cells without the immune acquisition process based on the expression of the major histocompatibility complex (MHC), and in particular, can kill target cells that express little or no Class 1 MHC better. Therefore, NK cells can effectively kill most cancer cells that do not express MHC, and in addition, they can kill cells infected with some viruses and bacteria such as Salmonella typhi.

However, NK cells, which have a potent effect on killing cancer cells, account for only 5 to 15% of peripheral blood lymphocytes even in normal people, and decrease to less than 1% of peripheral blood lymphocytes especially in cancer patients. Therefore, there is a limit to effectively attacking cancer cells without a separate amplification process through immunotherapy.

Cytokines such as IL-2 and antibodies such as CD3 antibodies are used to activate and proliferate immune cells, especially NK cells. In the current technology, CD3 antibodies play a very important role in the proliferation of immune cells. There is a problem in that it is very difficult to activate immune cells using this antibody. In other words, the current general technology for culturing immune cells mainly uses immobilization of CD3 antibodies on a flask and stimulating the antibody for a predetermined period of time. However, immune cells have different sensitivities, and significantly depend on cell culture conditions or the skill of the operator.

In particular, immature precursor cells mature into T cells when CD3 antibodies are strongly stimulated from the beginning. Therefore, in general, CD3 antibodies are slightly stimulated at the beginning. However, it is difficult to obtain a large amount of activated NK cells that are proliferated due to the surrounding environmental conditions such as differences in subjects.

There is an increasing need to develop a novel culture medium composition and culture method that are capable of stably amplifying and mass-proliferating NK cells while eliminating the inconvenience of the current method, such as the necessity of immobilizing CD3 antibodies in a flask and then reacting the same for a predetermined period of time.

The general technology for culturing immune cells, especially natural killer cells, which have been developed so far, involves incorporation of feeder cells, most of which are cancer cells, and is based on a method in which these immune cells and natural killer cells recognize cancer cells and increase their proliferation signals. This method has the problem of the difficulty in proving that all of the incorporated cancer cells have died. Therefore, there is a need for a novel effective method for culturing immune cells and natural killer cells.

### [Disclosure]

### [Technical Problem]

Therefore, the present invention has been made in view of the above problems, and it is an object of the present invention to provide a dual culture method that is capable of increasing the interaction between the natural killer cells and more effectively and stably increasing the number of cells by culturing immune cells, such as natural killer cells, under different stimulation conditions for a predetermined period of time and then combining the immune cells.

The objects of the present invention are not limited to those described above. The objects of the present invention will be clearly understood from the following description and are capable of being implemented by means defined in the claims and combinations thereof.

### [Technical Solution]

In accordance with the present invention, the above and other objects can be accomplished by the provision of a dual culture method in which immune cells (PBMC) or natural killer cells (NK cells) separated from blood are divided into groups, different stimuli are applied to the respective groups for a predetermined period of time during the culture period, and the groups are then cultured in combination. That is, the dual culture method is characterized by a method in which the immune cells to be cultured are divided into two or more groups in the Initial step of culture, the groups are stimulated with different antibodies or cytokines for a predetermined period of time during the Initial step of culture, and the groups are cultured in combination, and a method in which the immune cells to be cultured are divided into two or more groups in the intermediate step of culture, the groups are stimulated with different antibodies or cytokines for a predetermined period of time during the intermediate step of culture, and the groups are cultured in combination. Such a dual method is capable of more effectively proliferating immune cells and natural killer cells than a general culture method.

A dual culture method for immune cells including sequentially performing initial culture, proliferation culture, and mass culture of natural killer cells isolated from lymphocytes or peripheral blood mononuclear cells (PBMC) separated from blood and the method includes dividing immune cells into at least two groups, stimulating the cells of the respective groups with different combinations of antibodies or cytokines for a predetermined period of time during an immune cell culture period, and culturing the cells of the groups in combination during the next culture period.

The method may include dividing immune cells into at least two groups in an initial culture step, stimulating the cells of the respective groups with different combinations of antibodies or cytokines for an initial culture period of time and culturing the cells of the groups in combination.

The method may include dividing immune cells into at least two groups in a proliferation culture step, stimulating the cells of the respective groups with different combinations of antibodies or cytokines for a proliferation culture period of time and culturing the cells of the groups in combination.

The initial culture may be performed for 4 to 6 days, and the proliferation culture may be performed for 4 to 6 days.

The medium used for the dual culture may contain a BM solution containing L-glutamine, IL-2, and a medium for cell culture, a medium containing cytokines, and a medium containing antibodies, the medium containing cytokines may contain a C2 solution containing IL-2 and having a higher IL-2 content than the BM solution, a C12 solution containing IL-12, a C15 solution containing IL-15, a C17 solution containing IL-17, a C18 solution containing IL-18, and a C21 solution containing IL-21, and the medium containing antibodies may contain an A3 solution containing anti-CD3 antibodies, an A16 solution containing anti-CD16 antibodies, and an A56 solution containing anti-CD56 antibodies.

To provide different antibody stimulation in the initial culture step, the method may include equally dividing lymphocytes or natural killer cells (NK cells) into at least two groups, adding an A3 solution to a culture medium of one group containing a BM solution and two or more of a C2 solution, a C12 solution, a C15 solution, a C17 solution, a C18 solution, and a C21 solution, and adding an A16 solution and an A56 solution to the culture medium of another group, and stimulating the groups with different antibodies, and culturing the cells in combination.

To provide different antibody stimulation in the initial culture step, the method may include equally dividing lymphocytes or natural killer cells (NK cells) into at least two groups, adding an A3 solution and an A16 solution to a culture medium of one group containing a BM solution and two or more of a C2 solution, a C12 solution, a C15 solution, a C17 solution, a C18 solution, and a C21 solution, and adding an A3 solution and an A56 solution to the culture medium of another group, and stimulating the groups with different antibodies, and culturing the cells in combination.

To provide different cytokine stimulation in the initial culture step, the method may include equally dividing lymphocytes or natural killer cells (NK cells) into at least two groups, adding different combinations of a C2 solution, a C12 solution, a C15 solution, a C17 solution, a C18 solution, and a C21 solution to a medium containing a BM solution and at least one of an A3 solution, an A16 solution, or an A56 solution to two groups, stimulating the cells of the respective groups with different cytokines, and culturing the cells in combination.

To provide different antibody stimulation in the proliferation culture step, the method may include equally dividing lymphocytes or natural killer cells (NK cells) into at least two groups, adding an A16 solution to a culture medium of one group containing a BM solution and two or more of a C2 solution, a C12 solution, a C15 solution, a C17 solution, a C18 solution, and a C21 solution, and adding an A56 solution to the culture medium of another group, and stimulating the groups with different antibodies, and culturing the cells in combination.

To provide different cytokine stimulation in the proliferation culture step, the method may include equally dividing lymphocytes or natural killer cells (NK cells) into at least two groups, adding different combinations of a C2 solution, a C12 solution, a C15 solution, a C17 solution, a C18 solution, and a C21 solution to a medium containing a BM solution and at least one of an A3 solution, an A16 solution, or an A56 solution to two groups, and stimulating the cells with different cytokines, and culturing the cells in combination.

The BM solution may contain IL-2 at a concentration of 100 to 2,000 IU/mL, the C2 solution may contain IL-2 at a concentration of 250 to 4,000 IU/mL, the C12 solution may contain IL-12 at a concentration of 0.01 to 10 ug/mL, the C15 solution may contain IL-15 at a concentration of 0.01 to 10 ug/mL, the C17 solution may contain IL-17 at a concentration of 0.01 to 10 ug/mL, the C18 solution may contain IL-18 at a concentration of 0.01 to 10 ug/mL, the C21 solution may contain IL-21 at a concentration of 0.01 to 10 ug/mL, the A3 solution may contain anti-CD3 antibodies at a concentration of 0.1 to 20 ug/mL, the A16 solution may contain anti-CD16 antibodies at a concentration of 0.1 to 20 ug/mL, and the A56 solution may contain anti-CD56 antibodies at a concentration of 0.1 to 20 ug/mL.

The method may include (1) stimulating natural killer cells (NK cells) separated from blood or peripheral blood mononuclear cells (PBMCs) with a medium additive kit for culturing immune cells collected from blood or natural killer cells collected from blood, containing a BM solution and an A3 solution, an A16 solution, and an A56 solution, further containing two or more of a C12 solution, a C15 solution, a C17 solution, a C18 solution, and a C21 solution, and further containing autologous plasma, (2) separating the medium used in step 1 to stabilize the cells, (3) accelerating proliferation of lymphocytes or natural killer cells containing two or more of the C2 solution, the C12 solution, the C15 solution, the C17 solution, the C18 solution, and the C21 solution, and supplementing with the autologous plasma, and (4) mass-proliferatively culturing lymphocytes or natural killer cells in the medium including the BM solution and autologous plasma.

In addition, the autologous plasma may be added at 10% v/v or less of the total medium, and may be obtained by separating lymphocytes from blood cells or may be commercially available FBS or the like.

In addition, when immune cells (PBMC) or natural killer cells are cultured, the number of immune cells is increased 100 to 1,000 times the initial number of lymphocytes of 0.30 to 2.0x10⁷.

In accordance with another aspect of the present invention, provided is a pharmacological composition containing immune cells cultured by the dual culture method described above.

In addition, the pharmacological composition may prevent or treat at least one disease among blood cancer, cancer including solid cancer, viral infection, and autoimmune disease.

### [Advantageous effects]

The dual culture method according to the present invention is capable of increasing the interaction between the natural killer cells and more effectively and stably increasing the number of cells by culturing immune cells, such as natural killer cells, under different stimulation conditions for a predetermined period of time and then combining the immune cells.

The effects of the present invention are not limited to those mentioned above. It should be understood that the effects of the present invention include all effects that can be inferred from the description of the present invention.

### [Description of Drawings]

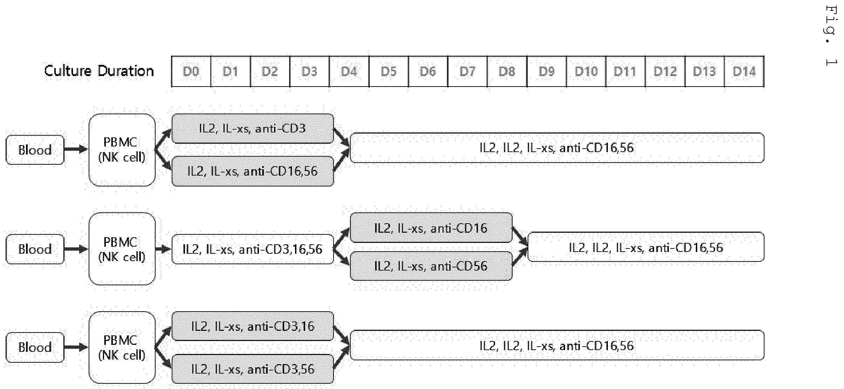

FIG. 1 illustrates a protocol for immune cell dual culture according to the present invention.

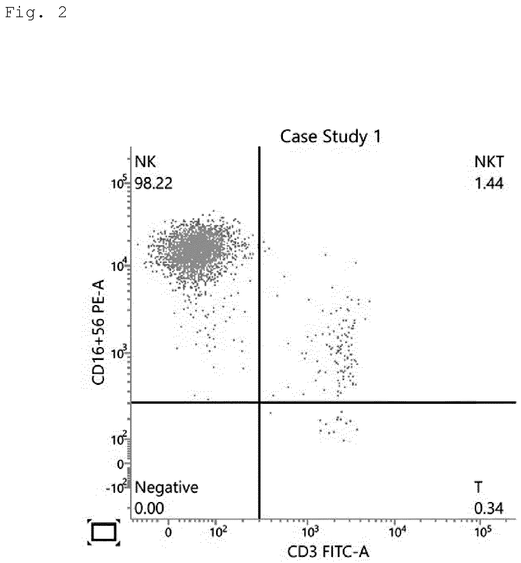

FIG. 2 is a graph showing the results of the natural killer cell ratio according to the immune cell dual culture method A according to the present invention.

### [Best Mode]

In the following description of the present invention, detailed descriptions of known functions and configurations incorporated herein will be omitted when the same may obscure the subject matter of the present invention. The terms which will be described below are defined in consideration of functions in the present invention and may be changed depending on intentions of users or judicial precedents, and thus the definitions should be understood based on the contents throughout the specification for describing the present invention.

### [Example 1] Preparation of medium (culture kit) for culture

100 mL of a 200 mM L-glutamine solution was dissolved in a basic medium for culturing suspension cells, IL-2 was added to reach 250 IU/mL, and a final volume of 10 L was obtained which was used as a BM solution.

IL-2 was dissolved at a concentration of 1,000 IU/mL in a BM solution to prepare a C2 solution.

IL-12 was dissolved at a concentration of 1 ug/mL in a BM solution to prepare a C12 solution.

IL-15 was dissolved at a concentration of 1 ug/mL in a BM solution to prepare a C15 solution.

IL-17 was dissolved at a concentration of 1 ug/mL in a BM solution to prepare a C17 solution.

IL-18 was dissolved at a concentration of 1 ug/mL in a BM solution to prepare a C18 solution.

IL-21 was dissolved at a concentration of 1 ug/mL in a BM solution to prepare a C21 solution.

An anti-CD3 antibody was dissolved at a concentration of 1 ug/mL in a BM solution to prepare an A3 solution.

An anti-CD16 antibody was dissolved at a concentration of 1 ug/mL in a BM solution to prepare an A16 solution.

An anti-CD56 antibody was dissolved at a concentration of 1 ug/mL in a BM solution to prepare an A56 solution.

### [Example 2] Single culture method

Lymphocytes and autologous plasma were prepared from blood of a patient and then lymphocytes containing natural killer cells were cultured using the cell culture medium addition kit prepared in Example 1 as follows.

### 1. Lymphocyte extraction and autologous plasma preparation

30 mL of peripheral blood of patient A was injected into a 50 mL conical tube and centrifuged, the autologous plasma of the upper layer was placed in a heparin tube and treated, injected into a novel 50 mL conical tube and centrifuged again, and the plasma component of the upper layer was prepared as autologous plasma.

Then, the blood tube from which the plasma was removed was filled with PBS to obtain a volume of 30 ml, thoroughly mixed, transferred to a tube containing the prepared Ficoll-Paque Plus, centrifuged at 800 x g for 15 minutes, the second layer, the buffy coat layer containing lymphocytes, was separated and collected in a 50 ml conical tube, and the volume was adjusted to 50 ml with PBS and then mixed. Then, the supernatant was discarded through 2 to 3 centrifugations to separate the lymphocytes.

### 2. Step 1: Initial Culture

A BM solution, a C12 solution, a C18 solution, an A3 solution, an A16 solution, an A56 solution, and an autologous plasma were added to 0.30x10⁷ to 2.0x10⁷ cells taken from the separated lymphocytes and initial culture was performed for 4 days, from day 1, the day when culture began, to day 5, in a T25 or T75 flask in a CO₂ incubator (37°C 5% CO₂).

### 3. Step 2: Stabilization

After the initial culture, on the 5th day of culture, the culture flask was scraped, transferred to a conical tube, centrifuged (400g, 5 min) in PBS, washed once more with PBS, and then transferred to a T75 flask using the culture solution containing the BM solution and C2 solution and stabilized.

### 4. Step 3: Proliferation culture

The cells stabilized in the T75 flask were added with a solution containing a C2 solution, a C12 solution, a C15 solution, a C17 solution, a C18 solution, a C21 solution, and a culture medium containing an A16 solution and an A56 solution, and continuously cultured in a CO2 incubator (37°C 5% CO₂) from the 5th to the 6th day of culture. As the number of cells increased, on the 7th day of culture, the cells were transferred to a T150 or T175 flask using a solution containing a C2 solution, a C12 solution, a C15 solution, a C17 solution, a C18 solution, and a C21 solution, and a culture medium containing an A16 solution and an A56 solution, and the culture medium was continuously added until the 8th day, and continuously cultured in a CO₂ incubator (37 °C 5% CO₂). (a total of 4 days)

### 5. Step 4: Mass culture

After step 3 of proliferation culture, the entire culture product was injected into a 1L CO₂-permeable culture bag containing the cell culture medium, 5 to 10 mL of autologous plasma was added thereto, and the mixture was massaged to mix well with the culture medium and mass culture was performed in a CO₂ incubator (37°C 5% CO₂) for 5 days. The culture medium was massaged every 24 hours to mix well.

### 6. Harvesting of natural killer immune cells

The final culture medium after culture was placed in a centrifuge tube and the cells were harvested through several centrifugations, and the harvested cells were packaged in a saline bag and stored in the refrigerator or frozen.

### [Example 3] Dual culture method (A) Initial step dual culture

In the initial culture step 1 in the single culture method of Example 2, the prepared cells were divided equally into two halves with the same cell number and cultured in two flasks. At this time, the medium contained the BM solution, the C12 solution, and the C18 solution in common, the A3 solution was added to one flask, and the A16 solution and the A56 solution were added to the other flask, followed by separate culture, stabilization of step 2 and combination culture in the proliferation culture of step 3.

### [Example 4] Dual culture method (B) Initial step dual culture

In the initial culture step 1 in the single culture method of Example 2, the prepared cells were divided equally into two halves with the same cell number and cultured in two flasks. At this time, the medium contained the BM solution, the C12 solution, and the C18 solution in common, the A3 solution and the A16 solution were added to one flask, and the A3 solution and the A56 solution were added to the other flask, followed by separate culture, stabilization of step 2 and combination culture in the proliferation culture of step 3.

### [Example 5] Dual culture method (C) Intermediate step dual culture

In the proliferation culture step 3 in the single culture method of Example 2, the cultured cells stabilized in step 2 were divided equally into two halves with the same cell number and cultured in two flasks. At this time, the medium contained the BM solution, the C2 solution, the C12 solution, the C15 solution, the C18 solution and the C21 solution in common, the A16 solution was added to one flask, and the A56 solution were added to the other flask, followed by separate culture and then combination culture in the mass culture of step 4.

### [Example 6] Comparison of single culture method with general culture method

The culture method using Example 2 was compared with the culture method using cell mass culture medium from KOHJIN BIO. The culture was compared using KOHJIN BIO's NK Kit (NKCC-1, NKCC-2, NKCC-b, NKCC-c), known as an NK cell culture kit for 14 days. The result showed that Example 2 exhibited higher immune cell count and natural killer cell ratio. The results are shown in Table 1 below.

**[Table 1]**

| Culture method | Subject | Immune cell count before culture | Immune cell count before culture | NK cell rate |
|---|---|---|---|---|
| KOHJIN BIO KIT | Healthy | 0.41 x 10⁷ | 126 x 10⁷ | 32% |
| | Patient | 0.42 x 10⁷ | 88 x 10⁷ | 24% |
| Example 2 method | Healthy | 0.40 x 10⁷ | 448 x 10⁷ | 98% |
| | Patient | 0.38 x 10⁷ | 413 x 10⁷ | 92% |

### [Example 7] Comparison of the number of cultured cells and anticancer activity of the single culture method and the dual culture method

The single culture method of Example 2 was compared with the dual culture methods of Examples 3, 4, and 5. The result showed that the number of cells in the dual culture method was higher than that in the single culture. Meanwhile, the K562 cancer cell line was stained with Cacein AM, immune cells or natural killer cells were added at a ratio of 1:30 and reacted in a CO₂ incubator (37°C, 5% CO₂) for 4 hours, and anticancer activity was analyzed by fluorescence colorimetry. At this time, all the methods exhibited high anticancer activity of more than 90% and the results are shown in Table 2 below.

**[Table 2]**

| Item | Initial immune cell count | Immune cell count before culture | Cytotoxicity (K562) |
|---|---|---|---|
| Single culture method | 0.44 x 10⁷ | 460 x 10⁷ | 94% |
| Dual culture method A | 0.46 x 10⁷ | 476 x 10⁷ | 98% |
| Dual culture method B | 0.45 x 10⁷ | 478 x 10⁷ | 95% |
| Dual culture method C | 0.45 x 10⁷ | 522 x 10⁷ | 96% |

### [Example 8] Analysis of immune cell rates from PBMCs using single culture method and dual culture method

When the cells are cultured using PBMCs isolated from blood, the ratio of proliferated immune cell subtypes may vary. Blood was collected from several donors who agreed to participate in the study, and PBMCs were isolated and cultured using different culture methods, namely, single culture method, dual culture method A, dual culture method B, and dual culture method C. The cultured cells were analyzed by FACS. In this case, the dual culture method exhibited a higher natural killer cell ratio. In particular, the initial culture of Step 1 in dual culture methods A and B exhibited a higher NK content than the single culture method, and the results are shown in Table 3 below.

**[Table 3]**

| Item | Culture method | NK cell rate before culture | NK cell rate after culture |
|---|---|---|---|
| Subject 1 | Single culture method | 11.0% | 88% |
| | Dual culture method A | | 98% |
| | Dual culture method B | | 95% |
| | Dual culture method C | | 90% |
| Subject 2 | Single culture method | 7.8% | 76% |
| | Dual culture method A | | 94% |
| | Dual culture method B | | 91% |

[Example 9] Changes in the number of cultured cells of NK cells isolated from PBMC using single culture method and dual culture method

Natural killer cells were cultured as follows using the cell culture medium addition kit prepared in Example 1.

### 1. Lymphocyte extraction and autologous plasma preparation

30 mL of peripheral blood of patient A was injected into a 50 mL conical tube and centrifuged, the autologous plasma of the upper layer was placed in a heparin tube and treated, injected into a novel 50 mL conical tube and centrifuged again, and the plasma component of the upper layer was prepared as autologous plasma.

Only natural killer cells (NK cells) with a purity of 99.9% were isolated from peripheral blood mononuclear cells (PBMCs) separated from blood using an NK isolation kit (STEM Cell).

### 2. Step 1: Initial Culture

A BM solution, a C12 solution, a C18 solution, an A3 solution, an A16 solution, an A56 solution, and an autologous plasma were added to 0.30x10⁷ to 2.0x10⁷ cells taken from the separated natural killer cells and initial culture was performed for 4 days, from day 1, the day when culture began, to day 5, in a T25 or T75 flask in a CO₂ incubator (37°C 5% CO₂).

### 3. Step 2: Stabilization

After the initial culture, on the 5th day of culture, the culture flask was scraped, transferred to a conical tube, centrifuged (400g, 5 min) in PBS, washed once more with PBS, and then transferred to a T75 flask using the culture solution containing the BM solution and C2 solution and stabilized.

### 4. Step 3: Proliferation culture

The cells stabilized in the T75 flask were added with a solution containing a C2 solution, a C12 solution, a C15 solution, a C17 solution, a C18 solution, a C21 solution, and a culture medium containing an A16 solution and an A56 solution, and continuously cultured in a CO2 incubator (37°C 5% CO₂) from the 5th to the 6th day of culture. As the number of cells increased, on the 7th day of culture, the cells were transferred to a T150 or T175 flask using a solution containing a C2 solution, a C12 solution, a C15 solution, a C17 solution, a C18 solution, and a C21 solution, and a culture medium containing an A16 solution and an A56 solution, and the culture medium was continuously added until the 8th day, and continuously cultured in a CO₂ incubator (37°C 5% CO₂). (a total of 4 days)

### 5. Step 4: Mass culture

After step 3 of proliferation culture, the entire culture product was injected into a 1L CO₂-permeable culture bag containing the cell culture medium, 5 to 10 mL of autologous plasma was added thereto, and the mixture was massaged to mix well with the culture medium and mass culture was performed in a CO₂ incubator (37°C 5% CO₂) for 5 days. The culture medium was massaged every 24 hours to mix well.

### 6. Harvesting of natural killer cells

The final culture medium after culture was placed in a centrifuge tube and the cells were harvested through several centrifugations, and the harvested cells were packaged in a saline bag and stored in the refrigerator or frozen.

### [Example 10] Dual culture method (A) Initial step dual culture

In the initial culture step 1 in the single culture method of Example 9, the prepared cells were divided equally into two halves with the same cell number and cultured in two flasks. At this time, the medium contained the BM solution, the C12 solution, and the C18 solution in common, the A3 solution was added to one flask, and the A16 solution and the A56 solution were added to the other flask, followed by separate culture, stabilization of step 2 and combination culture in the proliferation culture of step 3.

### [Example 11] Dual culture method (B) Initial step dual culture

In the initial culture step 1 in the single culture method of Example 9, the prepared cells were divided equally into two halves with the same cell number and cultured in two flasks. At this time, the medium contained the BM solution, the C12 solution, and the C18 solution in common, the A3 solution and the A16 solution were added to one flask, and the A3 solution and the A56 solution were added to the other flask, followed by separate culture, stabilization of step 2 and combination culture in the proliferation culture of step 3.

### [Example 12] Dual culture method (C) Intermediate step dual culture

In the proliferation culture step 3 in the single culture method of Example 2, the cultured cells stabilized in step 2 were divided equally into two halves with the same cell number and cultured in two flasks. At this time, the medium contained the BM solution, the C2 solution, the C12 solution, the C15 solution, the C18 solution and the C21 solution in common, the A16 solution was added to one flask, and the A56 solution were added to the other flask, followed by separate culture and then combination culture in the mass culture of step 4.

When natural killer cells were cultured using the single culture method of Example 9 and the dual culture methods of Examples 10 to 12, the dual culture method A exhibited a higher culture efficiency and the results are shown in Table 4 below.

**[Table 4]**

| Item | Initial NK cell count | NK cell count after culture |
|---|---|---|
| Single culture method | 0.041 x 10⁷ | 5.14 x 10⁷ |
| Dual culture method A | 0.040 x 10⁷ | 8.88 x 10⁷ |
| Dual culture method B | 0.040 x 10⁷ | 8.68 x 10⁷ |
| Dual culture method C | 0.040 x 10⁷ | 9.24 x 10⁷ |

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### [Industrial Applicability]

The novel dual culture method for culturing immune cells and natural killer cells of the present invention and the use thereof have industrial applicability.

## Claims

1. A dual culture method for immune cells including lymphocytes or natural killer cells comprising:
dividing immune cells into at least two groups;
stimulating the cells of the respective groups with different combinations of antibodies or cytokines for a predetermined period of time during an immune cell culture period; and
culturing the cells of the groups in combination during a next culture period.

2. The dual culture method according to claim 1, wherein the medium used for the dual culture comprises:
a BM solution containing L-glutamine, IL-2, and a medium for cell culture;
a medium containing cytokines; and
a medium containing antibodies,
the medium containing cytokines comprises:
a C2 solution containing IL-2 and having a higher IL-2 content than the BM solution;
a C12 solution containing IL-12;
a C15 solution containing IL-15;
a C17 solution containing IL-17;
a C18 solution containing IL-18; and
a C21 solution containing IL-21, and
the medium containing antibodies comprises:
an A3 solution containing anti-CD3 antibodies;
an A16 solution containing anti-CD16 antibodies; and
an A56 solution containing anti-CD56 antibodies.

3. The method according to claim 1, comprising:
equally dividing lymphocytes or natural killer cells (NK cells) into at least two groups,
adding different combinations of a C2 solution, a C12 solution, a C15 solution, a C17 solution, a C18 solution, and a C21 solution to a medium containing a BM solution and at least one of an A3 solution, an A16 solution, or an A56 solution to two groups to stimulate the cells with different cytokines; and
culturing the cells in combination.

4. The method according to claim 1, comprising:
equally dividing lymphocytes or natural killer cells (NK cells) into at least two groups;
adding at least one of an A3 solution, an A16 solution, or an A56 solution to a culture medium of one group containing a BM solution and two or more of a C2 solution, a C12 solution, a C15 solution, a C17 solution, a C18 solution, and a C21 solution, and adding at least one of an A3 solution, an A16 solution, and an A56 solution to the culture medium of another group, and stimulating the solutions added to the two groups with different combinations of solutions containing different antibodies; and
culturing the cells in combination.

5. The method according to claim 1, wherein the BM solution contains IL-2 at a concentration of 100 to 2,000 IU/mL, the C2 solution contains IL-2 at a concentration of 250 to 4,000 IU/mL, the C12 solution contains IL-12 at a concentration of 0.01 to 10 ug/mL, the C15 solution contains IL-15 at a concentration of 0.01 to 10 ug/mL, the C17 solution contains IL-17 at a concentration of 0.01 to 10 ug/mL, the C18 solution contains IL-18 at a concentration of 0.01 to 10 ug/mL, the C21 solution contains IL-21 at a concentration of 0.01 to 10 ug/mL, the A3 solution contains anti-CD3 antibodies at a concentration of 0.1 to 20 ug/mL, the A16 solution contains anti-CD16 antibodies at a concentration of 0.1 to 20 ug/mL, and the A56 solution contains anti-CD56 antibodies at a concentration of 0.1 to 20 ug/mL.

6. The method according to claim 1, comprising:
(1) stimulating natural killer cells (NK cells) separated from blood or peripheral blood mononuclear cells (PBMCs) with a medium additive kit for culturing immune cells collected from blood or natural killer cells collected from blood, containing a BM solution and an A3 solution, an A16 solution, and an A56 solution, further containing two or more of a C12 solution, a C15 solution, a C17 solution, a C18 solution, and a C21 solution, and further containing autologous plasma;
(2) separating the medium used in step 1 to stabilize the cells;
(3) accelerating proliferation of lymphocytes or natural killer cells containing two or more of the C2 solution, the C12 solution, the C15 solution, the C17 solution, the C18 solution, and the C21 solution, and supplementing with the autologous plasma; and
(4) mass-proliferatively culturing lymphocytes or natural killer cells in the medium including the BM solution and autologous plasma.
